(19) Europäisches Patentamt
European Patent Office
Office européen des brevets

(11) **EP 3 167 858 A1**

(12) **EUROPEAN PATENT APPLICATION**

(43) Date of publication:
**17.05.2017 Bulletin 2017/20**

(51) Int Cl.:
**A61F 13/532** (2006.01)   **A61F 13/534** (2006.01)
**A61L 15/60** (2006.01)   **A61F 13/53** (2006.01)

(21) Application number: **15194750.4**

(22) Date of filing: **16.11.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **The Procter & Gamble Company
Cincinnati, OH 45202 (US)**

(72) Inventor: **Kamphus, Juliane
65824 Schwalbach (DE)**

(74) Representative: **Heide, Ute
Procter & Gamble Service GmbH
IP Department
Frankfurter Strasse 145
61476 Kronberg im Taunus (DE)**

(54) **ABSORBENT CORES HAVING MATERIAL FREE AREAS**

(57)     An absorbent core for use in an absorbent article is provided and comprises a core wrap enclosing an absorbent material, the absorbent material comprising superabsorbent polymer particles. The core wrap comprises a top side and a bottom side. The absorbent core comprises one or more area(s) substantially free of absorbent material through which the top side of the core wrap is attached to the bottom side of the core wrap (i.e. core wrap bond(s)), so that when the absorbent material swells, the core wrap forms one or more channel(s) along the area(s) substantially free of absorbent material. The superabsorbent polymer particles have a value of Effective Capacity (EFFC) of at least 27 g/g. The Effective Capacity (EFFC) is calculated via the formula below: EFFC = (CRC+AAP)/2. The Centrifuge Retention Capacity (CRC) is measured according to the Centrifuge Retention Capacity (CRC) test method and the Absorption Against Pressure (AAP) is measured according to the Absorption Against Pressure (AAP) test method. The superabsorbent polymer particles have a bulk density of at least 0.5 g/ml according to the Bulk Density Test Method.

Fig. 1

**Description**

FIELD OF THE INVENTION

The invention provides absorbent cores for use in absorbent hygiene articles such as, but not limited to baby diapers, training pants, feminine hygiene sanitary pads and adult incontinence products.

BACKGROUND OF THE INVENTION

Absorbent articles for personal hygiene of the type indicated above are designed to absorb and contain body exudates, in particular large quantity of urine. The absorbent articles usually comprise several layers having different functions, for example a topsheet, a backsheet and in between an absorbent core, among other layers. The function of the absorbent core is to absorb and retain the exudates for a prolonged amount of time, for example, overnight for a diaper, minimize re-wet to keep the wearer dry and avoid soiling of clothes or bed sheets.

The majority of currently marketed absorbent articles comprise as absorbent material a blend of comminuted wood pulp fibers with superabsorbent polymers (SAP) in particulate form also called absorbent gelling materials (AGM), see for example US5151092 (Buell). Absorbent articles having a core consisting essentially of superabsorbent polymer particles as absorbent material (so called "airfelt-free" cores) have also been proposed (see e.g. WO2008/155699 (Hundorf), WO95/11652 (Tanzer), WO2012/052172 (Van Malderen)). Absorbent cores with slits or grooves have also been proposed, typically to increase the fluid acquisition properties of the core or to act as a folding guide.

WO2012/170778 (Rosati et al., see also WO2012/170779, WO2012/170781 and WO2012/170808) discloses absorbent structures that comprise superabsorbent polymer particles, optionally a cellulosic material, and at least a pair of substantially longitudinally extending channels. The core wrap can be adhesively bonded through the channels to form a channel bond. The channel bonds may be permanent, so that their integrity is at least partially maintained both in dry and wet state. As the absorbent structure absorbs liquid and swells, the absorbent structure takes a three-dimensional shape with the channels becoming visible. The channels are indicated to provide improved fit and/or better liquid acquisition/transportation, and/or improved performance throughout the use of the absorbent structure.

The properties of superabsorbent polymers have been characterized in various ways. The absorbent capacity (CRC) in grams of liquid per gram of superabsorbent particles has been used, as well as the absorption speed as measured by the Free Swell Rate (FSR) and their permeability as measured by the Urine Permeability Measurement (UPM) test

It has now been found that the creation of channels by bonding the core wrap may affect the performance of the absorbent core comprising superabsorbent polymer particles.

Therefore, there is a need for absorbent core having superabsorbent polymer particles that have special performance in order to overcome the potentially negative consequences of the creation of channels.

SUMMARY OF THE INVENTION

An absorbent core for use in an absorbent article is provided and comprises a core wrap enclosing an absorbent material, the absorbent material comprising superabsorbent polymer particles. The core wrap comprises a top side and a bottom side. The absorbent core comprises one or more area(s) substantially free of absorbent material through which the top side of the core wrap is attached to the bottom side of the core wrap (i.e. core wrap bond(s)), so that when the absorbent material swells, the core wrap forms one or more channel(s) along the area(s) substantially free of absorbent material. The superabsorbent polymer particles have a value of Effective Capacity (EFFC) of at least 27 g/g. The Effective Capacity (EFFC) is calculated via the formula below: EFFC = (CRC+AAP)/2. The Centrifuge Retention Capacity (CRC) is measured according to the Centrifuge Retention Capacity (CRC) test method as set out herein and the Absorption Against Pressure (AAP) is measured according to the Absorption Against Pressure (AAP) test method as set out herein. The superabsorbent polymer particles have a bulk density of at least 0.5 g/ml according to the Bulk Density Test Method.

The area(s) substantially free of absorbent material can be formed by various different means. For example, the top side of the core wrap can be attached to the bottom side of the core wrap, to form so called "core wrap bond(s)", via adhesive such as hot-melt adhesive. Alternatively or in addition, it is possible to bond via other known attachment means, such as pressure bonding, ultrasonic bonding, heat bonding or combination thereof. The area(s) substantially free of absorbent material can also be formed by cutting the areas out from the absorbent material (e.g. laying down a continuous layer of absorbent material and cutting out certain areas, thus forming areas substantially free of absorbent material).

The creation of core wrap bond(s) through area(s) substantially free of absorbent material can create tapered area(s) adjacent to the core wrap bond(s) i.e. the areas bordering the area(s) substantially free of absorbent material. The tapered area(s) results in a reduced space between the top and bottom side of the core wrap compared to other regions of the absorbent core which are more remote from the area(s) substantially free of absorbent material. Hence, in these tapered area(s), there is a reduced volume available to accommodate the absorbent material. However, the absorbent core has to contain a certain amount of absorbent material. This applies especially to the tapered area(s) bordering the area(s) substantially free of absorbent material since the area(s) substantially free of absorbent material constitute areas where no capacity to absorb fluid is available. In these tapered area(s), there is a need to provide a certain absorbent capacity per area in order to have leakage protection. Therefore, the required absorbent capacity of the absorbent material located in these tapered area(s) has to be provided in a relatively small volume. Moreover, the overall thickness of the absorbent core may be increased compared to an absorbent core having no area(s) substantially free of absorbent material, as the overall absorbent capacity of the absorbent core needs to be maintained to ensure proper liquid handling. Hence, the absorbent material which cannot be provided in the area(s) substantially free of absorbent material and in the tapered area(s) with limited space, need to be placed elsewhere in the absorbent core (i.e. in the areas surrounding the areas substantially free of absorbent material and the tapered area(s)).

The inventors have found that providing superabsorbent polymer particles having a relatively high value of Effective Capacity and a relatively high bulk density can help to improve the performance of the absorbent core comprising absorbent material such as superabsorbent polymer particles.

It has been found that when the superabsorbent polymer particles have a relatively high bulk density, the superabsorbent polymer particles are more densely packed. In combination with a relatively high value of Effective Capacity, the superabsorbent polymer particles take a smaller volume while still providing similar performance compared to superabsorbent polymer particles with a relatively low bulk density and a relatively low Effective Capacity. Thereby, having a superabsorbent polymer particles with a relatively high bulk density combined with a relatively high value of Effective Capacity (EFFC) enable to reduce the thickness of the absorbent core and enable to provide good performance of the absorbent core comprising absorbent material.

Having a reduced thickness of the absorbent core can bring to the wearer of the absorbent article some benefits especially in term of wearing confort and of discretness of the absorbent article. Moreover, when the absorbent core has a reduced thickness, the In-Bag Stack Height can be decreased without increasing the pressure inside the package of the absorbent article. Thus, the production of the package of the absorbent article may be improved in term of gain in productivity.

The value of EFFC maybe between 27 g/g and 32 g/g.

The bulk density of the superabsorbent polymer particles may be at least 0.6 g/ml.

The superabsorbent polymer particles may have a value of absorption against pressure (AAP) of at least 22 g/g.

The absorbent material may comprise at least 80%, in particular at least 90%, more particularly at least 95% of superabsorbent polymer particles by weight of the absorbent material.

The absorbent material may comprise less than 10% by weight of fibrous absorbent material, or less than 5% by weight of fibrous absorbent material, or may be substantially free of fibrous absorbent material.

The superabsorbent polymer particles may have a permeability at equilibrium expressed as UPM (Urine Permeability Measurement) value of more than 20 UPM units according to the UPM Test method.

The superabsorbent polymer particles may have a permeability at equilibrium expressed as UPM (Urine Permeability Measurement) value of more than 65 UPM units according to the UPM Test method.

At least one of the area(s) substantially free of absorbent material may have a width (Wc) in at least in some part of the area of at least 2 mm, in particular from 4 mm to 20 mm.

The core wrap may comprise a first nonwoven substantially forming the top side of the core wrap and a second nonwoven substantially forming the bottom side of the core wrap, preferably the first nonwoven forms a C-wrap around the second nonwoven.

The periphery of the absorbent material within the core wrap may define an absorbent material deposition area, and the absorbent material deposition area may be either rectangular or may be shaped with a width narrower at the crotch point (C') than the maximum width of the absorbent material deposition area in the rest of the core. The crotch point maybe defined as the point placed at a distance of two fifth (2/5) of L" from the front edge of the absorbent core on the longitudinal axis of the core.

The absorbent core may comprise a fibrous thermoplastic adhesive material, in particular a microfiber glue, to immobilize the absorbent material within the core.

The absorbent core may further comprise an auxiliary glue between the absorbent material and the top side and/or the bottom side of the core wrap.

The invention also relates to an absorbent article for personal hygiene comprising a liquid permeable topsheet, a liquid impermeable backsheet,optionally an acquisition and/or distribution layer, and an absorbent core as described herein between the topsheet and backsheet.

BRIEF DESCRIPTION OF THE DRAWINGS

Fig. 1 is a top view of an embodiment of an absorbent core according to the invention with the topside layer of the core wrap partially removed;
Fig. 2 is a transversal cross-section of the embodiment of Fig. 1 at the crotch point (C');
Fig. 3 is a close-up view of a part of Fig. 2;
Fig. 4 is a longitudinal cross-section of the embodiment of Fig. 1;
Fig. 5 is a close-up view of a part of Fig. 4;
Fig. 6 is a top view of an exemplary absorbent article in the form of a diaper with an absorbent core of the invention.
Fig. 7 is a transversal cross-section of the article of Fig. 6;
Fig. 8 is a transversal cross-section of the article taken at the same point as Fig. 7 where channels have formed in the core as a result of the diaper being loaded with fluid.
Fig. 9 is a schematic view of a package of absorbent articles of the present invention.

DETAILED DESCRIPTION OF THE INVENTION

## Introduction

As used herein, the term "Absorbent article" refers to devices that absorb and contain body exudates, and, more specifically, refers to devices that are placed against or in proximity to the body of the wearer to absorb and contain the various exudates discharged from the body. Absorbent articles may include diapers (baby diapers and diapers for adult incontinence), pants, inserts, feminine care absorbent articles such as sanitary napkins or pantiliners, and the like. As used herein, the term "exudates" includes, but is not limited to, urine, blood, vaginal discharges, sweat and fecal matter. Preferred absorbent articles of the present invention are disposable absorbent articles, more preferably disposable diapers and disposable pants. The absorbent articles of the invention will be further illustrated in the below description and in the Figures in the form of a disposable diapers. Nothing in this description should be however considered limiting the scope of the claims unless explicitly indicated otherwise.

"Disposable" is used in its ordinary sense to mean an article that is disposed or discarded after a limited number of usage over varying lengths of time, for example, less than 20 usages, less than 10 usages, less than 5 usages, or less than 2 usages. If the disposable absorbent article is a diaper, a pant, sanitary napkin, sanitary pad or wet wipe for personal hygiene use, the disposable absorbent article is most often intended to be disposed after single use.

"Diaper" and "pant" refers to an absorbent article generally worn by babies, infants and incontinent persons about the lower torso so as to encircle the waist and legs of the wearer and that is specifically adapted to receive and contain urinary and fecal waste. In a pant, the longitudinal edges of the first and second waist region are attached to each other to a pre-form waist opening and leg openings. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant may be preformed anywhere along the circumference of the article (e.g., side fastened, front waist fastened). In a diaper, the waist opening and leg openings are only formed when the diaper is applied onto a wearer by (releasably) attaching the longitudinal edges of the first and second waist region to each other on both sides by a suitable fastening system.

A "nonwoven web" as used herein means a manufactured sheet, web or batting of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yarn). Nonwoven webs can be formed by many processes such as meltblowing, spunbonding, solvent spinning, electrospinning, carding and airlaying. The basis weight of nonwoven webs is usually expressed in grams per square meter (g/m$^2$ or gsm).

"Comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

## General description of the absorbent core 28

The absorbent core of the invention will be typically made to be used in an absorbent article of the type indicated before such as a disposable diaper or a disposable pant. The absorbent core may for example be made on-line and assembled directly with the remaining components of the article or may be made off-line at another site and transported to the absorbent article manufacturing line. It is also possible to use the absorbent core directly as an absorbent article without further assembling of other components for applications which do not require other layers. Typically however the absorbent core will be assembled with other components such as a topsheet and a backsheet to form a finished absorbent article, as will be exemplary described further below for a disposable diaper or a disposable pant.

The absorbent core is typically the component of the article having the highest absorbent capacity. The absorbent core of the invention comprises a core wrap enclosing an absorbent material, and may also comprise at least one adhesive. The absorbent material comprises a superabsorbent polymer in particulate forms (herein abbreviated as "SAP") named "superabsorbent polymer particles". The absorbent material may comprise relatively high amount of superabsorbent polymer particles enclosed within the core wrap. By "absorbent material" it is meant a material which has some absorbency property or liquid retaining properties, such as SAP, cellulosic fibers as well as synthetic fibers. Typically, adhesives used in making absorbent cores have no absorbency properties and are not considered as absorbent material.

The SAP content may represent at least 80%, or at least 85%, or at least 90%, or at least 95% and up to 100% by weight of the absorbent material enclosed in the core wrap. The core wrap itself is not considered as absorbent material for the purpose of assessing the percentage of SAP in the absorbent core. High amount of SAP provides a relatively thin core compared to conventional core typically comprising between 40-60% by weight of cellulose fibers. The absorbent core may be thin, for example having a thickness not exceeding 5 mm, e.g. from 0.2 mm to 4 mm, in particular from 0.5 to 3 mm, as measured with the Dry Absorbent Core Caliper Test disclosed therein.

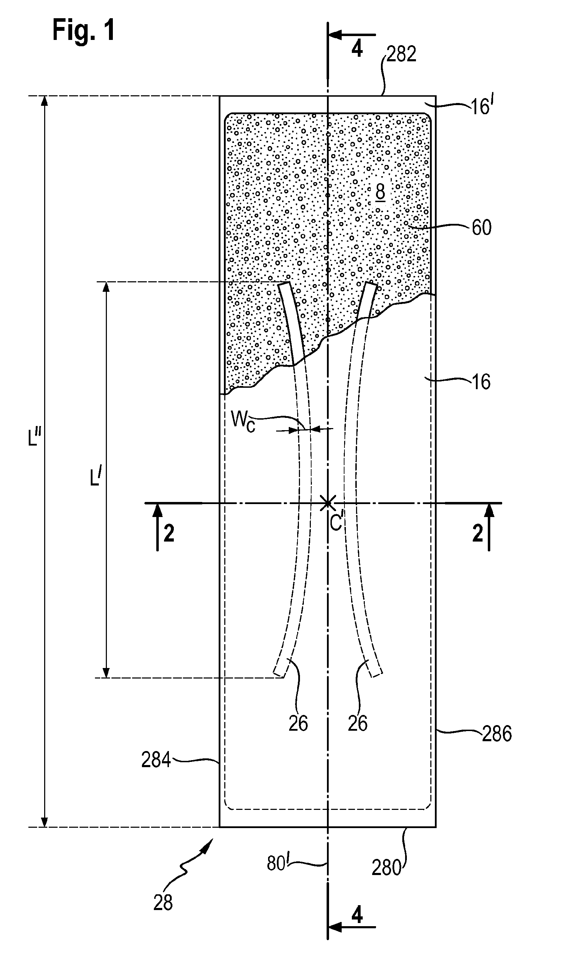

An exemplary absorbent core 28 of the invention is shown in isolation in Figs. 1-5 and will now be further described. The absorbent core shown and its description are purely for exemplary purpose and are not intended to limit the scope of the claims, unless otherwise stated. The absorbent core typically comprises a front side 280, a back side 282 and two longitudinal sides 284, 286 joining the front side 280 and the back side 282. The absorbent core also comprises a generally planar top side 16 and a generally planar bottom side 16' formed by the core wrap. The front side 280 of the core is the side of the core intended to be placed towards the front edge 10 of the absorbent article. The core may have a longitudinal axis 80' corresponding substantially to the longitudinal axis of the article 80, as seen from the top in a planar view as in Fig. 1. Typically the absorbent material will be advantageously distributed in higher amount towards the front side and middle portion of the core than towards the back side as more absorbency is required at the front. Typically the front and back sides of the core are shorter than the longitudinal sides of the core. The core wrap may be formed by two nonwoven materials which may be at least partially sealed along the sides of the absorbent core. The first nonwoven may substantially form the whole of the top side of the core wrap and the second nonwoven substantially the whole of the bottom side 16' of the core wrap. The top side and first nonwoven are represented by the same number 16 on the drawings, the bottom side and the second nonwoven by number 16'. The core wrap maybe at least partially sealed along its front side 280, back side 282 and/or two longitudinal sides 284, 286 to improve the containment of the absorbent material during use.

The absorbent core 28 comprises at least one area 26 which is substantially free of absorbent material and through which the top side of the core wrap is attached to the bottom side of the core wrap. When the absorbent material absorbs a liquid, it swells in proportion and the core wrap gradually forms a channel 26' along the bonded area 26 substantially free of absorbent material.

The length L" of the absorbent core as measured along it axis 80' from the front side 280 to the back side 282 should be adapted for the intended article in which it will be used. For infant diapers, the length L" may for example range from 5 to 40 cm. The absorbent core comprises a crotch point C' defined as the point on the longitudinal axis 80' situated at a distance of two fifth (2/5) of L" starting from the front side 280 of the absorbent core. The individual components of the absorbent core will now be described in further details.

## Core wrap (16, 16')

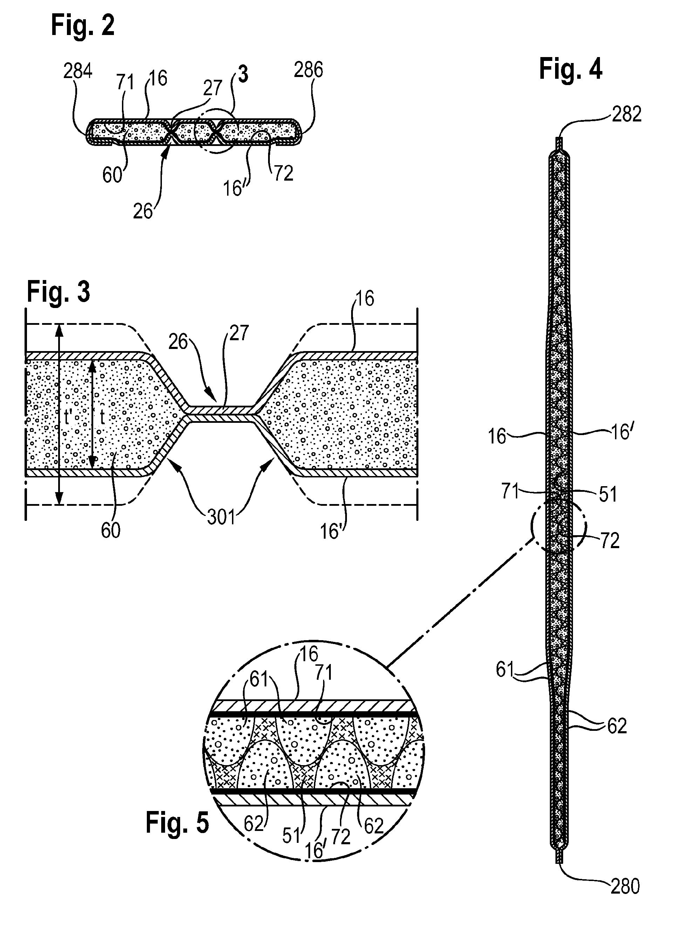

The function of the core wrap is to enclose the absorbent material. Typical core wraps comprise two substrates 16, 16' which are attached to another, but the core wrap may also be made of a single substrate folded around the absorbent material, or may comprises several substrates. When two substrates are used, these may be typically attached to another along at least part of the periphery of the absorbent core. Typical attachments are the so-called C-wrap and sandwich wrap. In a C-wrap, as exemplarily shown in Fig. 2, the longitudinal and/or transversal edges of one of the substrate are folded over the other substrate to form flaps. These flaps are then bonded to the external surface of the other substrate, typically by gluing. In a sandwich wrap, as shown on Fig. 4, the edges of both substrates are attached, e.g. by gluing, to another in a flat configuration.

The core wrap may be formed by any materials suitable for enclosing the absorbent material. Typical substrate materials used in the production of conventional cores may be used, in particular nonwovens but also paper, tissues, films, wovens, or laminate of any of these. The core wrap may in particular be formed by a nonwoven web, such as a carded nonwoven, a spunbond nonwoven ("S") or a meltblown nonwoven ("M"), and laminates of any of these. For example spunmelt polypropylene nonwovens are suitable, in particular those having a laminate web SMS, or SMMS, or SSMMS, structure, and having a basis weight range of about 5 gsm to 15 gsm. Suitable materials are for example disclosed in US7,744,576, US2011/0268932A1, US2011/0319848A1 or US2011/0250413A1. Nonwoven materials provided from synthetic fibers may be used, such as PE, PET and in particular PP.

If the core wrap comprises a first substrate 16 and a second substrate 16' these may be made of the same type of material, or may be made of different materials or one of the substrate may be treated differently than the other to provide it with different properties. As the polymers used for nonwoven production are inherently hydrophobic, they are preferably coated with hydrophilic coatings or otherwise rendered hydrophilic if placed on the fluid receiving side of the absorbent core. It is advantageous that the top side 16 of the core wrap, i.e. the side placed closer to the wearer in the absorbent article, be more hydrophilic than the bottom side 16' of the core wrap. A possible way to produce nonwovens with durably hydrophilic coatings is via applying a hydrophilic monomer and a radical polymerization initiator onto the nonwoven, and conducting a polymerization activated via UV light resulting in monomer chemically bound to the surface of the nonwoven. An alternative possible way to produce nonwovens with durably hydrophilic coatings is to coat the nonwoven with hydrophilic nanoparticles, e.g. as described in WO 02/064877.

Permanently hydrophilic nonwovens are also useful in some embodiments. Surface tension can be used to measure how permanently a certain hydrophilicity level is achieved. Liquid strike through can be used to measure the hydrophilicity level. The first and/or second substrate may in particular have a surface tension of at least 55, preferably at least 60 and most preferably at least 65 mN/m or higher when being wetted with saline solution. The substrate may also have a liquid strike through time of less than 5 seconds for a fifth gush of liquid. These values can be measured using the test methods described in US7,744,576B2 (Busam et al.): "Determination Of Surface Tension" and "Determination of Strike Through" respectively.

Hydrophilicity and wettability are typically defined in terms of contact angle and the strike through time of the fluids, for example through a nonwoven fabric. This is discussed in detail in the American Chemical Society publication entitled "Contact angle, wettability and adhesion", edited by Robert F. Gould (Copyright 1964). A substrate having a lower contact angle between the water and the surface of substrate may be said to be more hydrophilic than another.

The substrates may also be air-permeable. Films useful herein may therefore comprise micro-pores. The substrate may have for example an air-permeability of from 40 or from 50, to 300 or to 200 $m^3/(m^2 x min)$, as determined by EDANA method 140-1-99 (125 Pa, 38.3 $cm^2$). The material of the core wrap may alternatively have a lower air-permeability, e.g. being non-air-permeable, for example to facilitate handling on a moving surface comprising vacuum.

The core wrap may be sealed along its longitudinal edges and/or its transversal edges. In a C-wrap configuration, for example, a first substrate 16 maybe placed on one side of the core and extends around the core's longitudinal edges to partially wrap the opposed bottom side of the core (see Fig. 2). The second substrate 16' is typically present between the wrapped flaps of the first substrate 16 and the absorbent material 60. The flaps of the first substrate 16 may be glued to the second substrate 16' to provide a strong seal. This so called C-wrap construction can provide benefits such as improved resistance to bursting in a wet loaded state compared to a sandwich seal. The front side and back side of the core wrap may then also be sealed for example by gluing the first substrate and second substrate to another to provide complete enclosing of the absorbent material across the whole of the periphery of the core. For the front side and back side of the core the first and second substrate may extend and be joined together in a substantially planar direction, forming for these edges a so-called sandwich construction. In the so-called sandwich construction, the first and second substrates may also extend outwardly on all sides of the core and be sealed flat along the whole or parts of the periphery of the core typically by gluing and/or heat/pressure bonding. Typically neither first nor second substrates need to be shaped, so that they can be rectangularly cut for ease of production but of course other shapes are possible.

The terms "seal" and "enclosing" are to be understood in a broad sense. The seal does not need to be continuous along the whole periphery of the core wrap but may be discontinuous along part or the whole of it, such as formed by a series of seal points spaced on a line. Typically a seal may be formed by gluing and/or thermal bonding. The core wrap may also be formed by a single substrate which may enclose the absorbent material as in a parcel wrap and be for example sealed along the front side and back side of the core and one longitudinal seal.

**Absorbent material 60**

The absorbent core 28 comprises an absorbent material 60 comprising superabsorbent polymer particles ("SAP"). The absorbent material may be for example applied as a continuous layer. The absorbent material may also be comprised of individual pockets or stripes of absorbent material enclosed within the core wrap. A continuous layer of absorbent material, in particular of SAP, may also be obtained by combining two absorbent layers having matching discontinuous absorbent material application pattern wherein the resulting layer is substantially continuously distributed across the absorbent particulate polymer material area, as taught in US2008/0312622A1 (Hundorf) for example. In this way, each absorbent material layer comprises a pattern having absorbent material areas and absorbent material-free areas, wherein the absorbent material areas of the first layer correspond substantially to the absorbent material-free areas of the second layer and vice versa. A microfibrous glue 51 as disclosed further below maybe applied on each absorbent material layer to immobilize it on each substrate.

As exemplary shown in Figs. 4-5, the absorbent core 28 may thus comprise a first absorbent layer and a second absorbent layer, the first absorbent layer comprising a first substrate 16 and a first layer 61 of absorbent material, which may be 100% SAP, and the second absorbent layer comprising a second substrate 16' and a second layer 62 of absorbent material, which may also be 100% SAP. The first and second SAP layers may be applied as transversal stripes or "land areas" having the same width as the desired absorbent material deposition area 8 on their respective substrate before being combined. The stripes may advantageously comprise different amount of absorbent material to provide a profiled basis weight along the longitudinal axis and/or transversal axis of the core 80'. The first substrate 16 and the second substrate 16' may form the core wrap. An auxiliary glue 71, 72 may be applied between one or both substrates and the absorbent layers, as well as microfiber glue on each absorbent layer.

The absorbent material may comprise at least 80%, preferably at least 90%, more preferably at least 95% and up to 100% of superabsorbent polymer particles by weight of the absorbent material.

The absorbent material may also comprise fibrous absorbent material, such as natural fibers or synthetic fibers, or a combination thereof. Natural fibers may comprise cotton fibers or wood fibers such as eucalyptus fibers, acacia fibers, oak fibers, maple fibers or cherry fibers. Synthetic fibers may comprise polymeric fibers such as polyester, polypropylene or polyethylene fibers. Fibrous absorbent material excludes superabsorbent polymer particles in fibrous shape.

The fibrous absorbent material may comprise cellulosic material or modified cellulosic fibers. The cellulose fibers maybe partially or totally cross-linked.

Preferably, the absorbent material comprises fibrous absorbent material selected from natural fibers. Preferably, the natural fibers are wood fibers selected from cellulosic fibers or modified cellulosic fibers.

Preferably, the absorbent material comprises fibrous absorbent material selected from cellulosic fibers or modified cellulosic fibers.

At least 80% by weight of the fibrous absorbent material may be cellulosic fibers or modified cellulosic fibers based on the total weight of the fibrous absorbent material.

The absorbent material may consist of the superabsorbent polymer particles and fibrous absorbent material. The absorbent material may consist of the superabsorbent polymer particles and cellulosic material or modified cellulosic fibers.

The superabsorbent polymer particles and the fibrous absorbent material may be mixed with each other.

The fibrous absorbent material may serve the function of absorbing the fluid and spreading the liquid along the absorbent core 28.

The absorbent material may comprise less than 10% by weight of fibrous absorbent material, or less than 5% by weight of fibrous absorbent material, or may be substantially free of fibrous absorbent material.

The absorbent material may advantageously comprise little or no cellulosic fibers or modified cellulosic fibers, in particular the absorbent material may comprise less than 15% by weigth, preferably less than 10% by weigth, more preferably less than 5% by weigth of cellulosic fibers or modified cellulosic fibers based on the total weight of the absorbent material, or even be substantially free of cellulosic fibers or modified cellulosic fibers.

**Superabsorbent polymer particles (SAP)**

"Superabsorbent polymers" as used herein refer to absorbent material which are cross-linked polymeric materials that can absorb at least 10 times their weight of an aqueous 0.9% saline solution as measured using the Centrifuge Retention Capacity (CRC) test (EDANA method WSP 241.2-05). These polymers are typically used in particulate forms ("SAP") so as to be flowable in the dry state. The term "particles" refers to granules, fibers, flakes, spheres, powders, platelets and other shapes and forms known to persons skilled in the art of superabsorbent polymer particles.

Typical particulate absorbent polymer materials are made of poly(meth)acrylic acid polymers. However, e.g. starch-based particulate absorbent polymer material may also be used, as well polyacrylamide copolymer, ethylene maleic anhydride copolymer, cross-linked carboxymethylcellulose, polyvinyl alcohol copolymers, cross-linked polyethylene oxide, and starch grafted copolymer of polyacrylonitrile. The superabsorbent polymer may be polyacrylates and polyacrylic acid polymers that are internally and/ or surface cross-linked. The superabsorbent polymers can be internally cross-linked, i.e. the polymerization is carried out in the presence of compounds having two or more polymerizable groups which can be free-radically copolymerized into the polymer network. Exemplary superabsorbent polymer particles of the prior art are for example described in WO2006/083584, WO2007/047598, WO2007/046052, WO2009/155265, WO2009/155264.

The properties of superabsorbent polymers particles have been characterized in various ways.

The Centrifuge Retention Capacity (CRC) measures the liquid absorbed by the superabsorbent polymer particles for free swelling in excess liquid. The superabsorbent polymer particles may have a Centrifuge Retention Capacity (CRC) value of more than 18 g/g, or more than 20 g/g, or more than 22 g/g, or more than 24 g/g, for example up to 50 g/g, or up to 40 g/g, or to 30 g/g, as measured according to EDANA method WSP 241.2-05. Superabsorbent polymer particles having a high CRC value may be preferred since less superabsorbent polymer particles are needed to facilitate a required overall capacity for liquid absorption.

The Absorption Against Pressure (AAP) of superabsorbent polymers particles may be used to defined the performance of the absorbent core comprising superabsorbent polymer particles. The Absorption Against Pressure (AAP) of superabsorbent polymers particles corresponds to the capability of the superabsorbent polymers particles to swell against external pressure. The term "external pressure" refers to the pressure applied on the absorbent core by the wearer when he is seated for example or lay down and the pressure exerted by the core wrap bond(s).

The creation of core wrap bond(s) through area(s) substantially free of absorbent material can create tapered area(s) adjacent to the core wrap bond(s) i.e. the areas bordering the area(s) substantially free of absorbent material.

Fig. 3 illustrate a transversal cross-section of the absorbent core of the invention. In particular, Fig. 3 is a close-up view of a core wrap bond 27 and the area adjacent to the core wrap bond 27 i.e. the areas bordering the area 26 substantially free of absorbent material 60. The core wrap bond 27 may create tapered area(s) 301 adjacent to the core wrap bond 27 as represented on Fig. 3. The tapered area(s) 301 results in a reduced space between the top 16 and bottom side 16' of the core wrap compared to other regions of the absorbent core 28 which are more remote from the area 26 substantially free of absorbent material 60. Hence, in the tapered area(s) 301, there is reduced volume available to accommodate the absorbent material 60. However, the absorbent core 28 has to contain a certain amount of absorbent material 60. This applies especially to the tapered area(s) 301 bordering the area 26 substantially free of absorbent material since the area 26 substantially free of absorbent material constitute areas where no capacity to absorb fluid is available. In these tapered area(s) 301, there is a need to provide a certain absorbent capacity per area in order to have leakage protection. Therefore, the required absorbent capacity of the absorbent material 60 located in these tapered area(s) 301 has to be provided in a relatively small volume.

The inventors have found that providing superabsorbent polymer particles having a relatively high value of Absorption Against Pressure according to the Absorption Against Pressure Test Method can improve the performance of the absorbent core comprising absorbent material such as superabsorbent polymer particles.

The superabsorbent polymer particles may have a value of Absorption Against Pressure (AAP) of at least 22 g/g according to the Absorption Against Pressure Test Method.

The absorption against Pressure Test Method refers to the EDANA method WSP 442.2-02.

Preferably, the superabsorbent polymer particles have a value of Absorption Against Pressure (AAP) of at least 22.5 g/g, more preferably of at least 23 g/g, even more preferably of at least 23.5g/g, still preferably of at least 24 g/g, and most preferably of at least 24.5 g/g according to the Absorption Against Pressure Test Method.

The superabsorbent polymer particles have a relatively high value of Absorption Against Pressure (AAP) in order to allow the superabsorbent polymer particles to swell properly against pressure.

According to the invention, a parameter to defined the properties of superabsorbent polymer particles is used. It is called the Effective Capacity (EFFC). The Effective Capacity (EFFC) is calculated with the value of Centrifuge Retention Capacity (CRC) and with the value of Absorption Against Pressure (AAP) of the superabsorbent polymer particles. The Effective Capacity represents an average of the value of Centrifuge Retention Capacity (CRC) and of the value of Absorption Against Pressure (AAP) of the superabsorbent polymer particles.

The Effective Capacity (EFFC) is calculated via the formula below: EFFC = (CRC+AAP)/2.

According to the invention, the superabsorbent polymer particles have a value of Effective Capacity (EFFC) of at least 27 g/g.

Preferably, the superabsorbent polymer particles have a value of Effective Capacity (EFFC) of at least 27.5 g/g, more preferably of at least 28 g/g, even more preferably of at least 28.5 g/g and most preferably of at least 29 g/g.

The value of EFFC maybe between 27 g/g and 32 g/g.

According to the invention, the superabsorbent polymer particles have a bulk density of at least 0.5 g/ml according to the Bulk Density Test Method. The bulk density is defined as the amount of free flowing AGM that fits into a 100ml beaker.

The bulk density test method refers to the EDANA method WSP 460.2-02.

Preferably, the superabsorbent polymer particles have a bulk density of at least 0.55 g/ml, more preferably of at least 0.6 g/ml, even more preferably of at least 0.65 g/ml, and most preferably of at least 0.7 g/ml.

As shown on Fig. 3, the core wrap bond 27 may create tapered area(s) 301 adjacent to the core wrap bond 27. The overall thickness of the absorbent core 28 may be increased compared to an absorbent core having no area(s) substantially free of absorbent material, as the overall absorbent capacity of the absorbent core needs to be maintained to ensure proper liquid handling. Hence, the absorbent material 60 which cannot be provided in the area 26 substantially free of absorbent material and in the tapered area(s) 301 with limited space, need to be placed elsewhere in the absorbent core 28 (i.e. in the areas surrounding the areas substantially free of absorbent material and the tapered area(s)).

The inventors have found that providing superabsorbent polymer particles having a relatively high Effective Capacity and a relatively high bulk density can help to improve the performance of the absorbent core comprising absorbent material.

It has been found that when the superabsorbent polymer particles have a relatively high bulk density, the superabsorbent polymer particles are more densely packed. In combination with a relatively high value of Effective Capacity, the superabsorbent polymer particles take a smaller volume while still providing similar performance compared to superabsorbent polymer particles with a relatively low bulk density and a relatively low Effective Capacity.

As shown on Fig. 3, the dotted line represents the top side of the core wrap when the thickness of the absorbent core 28 is increased in the areas surrounding the area 26 substantially free of absorbent material and the tapered area(s) 301. The thickness t' is the thickness (caliper) of the absorbent core adjacent to the tapered area(s) 301 on the side of the tapered area(s) 301 which is opposite to the side where the core wrap bond 27 is provided when the superabsorbent polymer particles have a relatively low bulk density and a relatively low Effective Capacity.

The solid line represents the top side 16 of the core wrap when the thickness of the absorbent core 28 is reduced. The thickness t is the thickness (the caliper) of the absorbent core adjacent to the tapered area(s) 301 on the side of the tapered area(s) 301 which is opposite to the side where the core wrap bond 27 is provided when the superabsorbent polymer particles have a relatively high bulk density and a relatively high Effective Capacity.

When the superabsorbent polymer particles have a relatively high bulk density and a relatively high Effective Capacity according to the invention, the thickness t is lower than the thickness t'. Thus, the thickness (the caliper) of the absorbent core 28 is reduced when the superabsorbent polymer particles have a relatively high bulk density and a relatively high Effective Capacity according to the invention compared to superabsorbent polymer particles having a relatively low bulk density and a relatively low Effective Capacity.

Thereby, having a superabsorbent polymer particles with a relatively high bulk density combined with a relatively high value of Effective Capacity (EFFC) enable to reduce the thickness of the absorbent core and to provide good performance of the absorbent core comprising absorbent material.

Having a reduced thickness of the absorbent core can bring to the wearer of the absorbent article some benefits especially in term of wearing confort and of discretness of the absorbent article. Moreover, when the absorbent core has a reduced thickness, the In-Bag Stack Height can be decreased without increasing the pressure inside the package of the absorbent article. Thus, the production of the package of the absorbent article may be improved in term of gain in productivity.

The superabsorbent polymer particles may have a permeability at equilibrium expressed as UPM (Urine Permeability Measurement) value of more than 20, or preferably more than 40, or more than 60, or more than 70, or more than 80 UPM units, where 1 UPM unit is $1 \times 10^{-7}$ $(cm^3.s)$ /g. The UPM value is measured according to the UPM Test method set out in WO2012/174,026A1. The UPM Test method typically measures the flow resistance of a preswollen layer of superabsorbent polymer particles, i.e. the flow resistance is measured at equilibrium. Therefore, such superabsorbent polymer particles having a high UPM value exhibit a high permeability when a significant volume of the absorbent article is already wetted by the liquid exudates. These embodiments exhibit good absorption properties not only at the first gush but also at the subsequent gushes.

The total amount of superabsorbent polymer particles present in the absorbent core may also vary according to expected user of the article. Diapers for newborns require less superabsorbent polymer particles than infant or adult incontinence diapers. The amount of superabsorbent polymer particles in the core may be for example comprised from about 2 to 50 g, in particular from 5 to 40 g for typical enfant diapers or adult incontinence diapers. The average superabsorbent polymer particles basis weight within the (or "at least one", if several are present) deposition area 8 of the superabsorbent polymer particles may be for example of at least 50, 100, 200, 300, 400, 500 or more $g/m^2$. The material free areas 26 present in the absorbent material deposition area 8 are deduced from the absorbent material deposition area to calculate this average basis weight.

**Area(s) 26 substantially free of absorbent material and channels 26'**

The absorbent core 28 comprises one or more area(s) 26 which is/are substantially free of absorbent material. By "substantially free" it is meant that in each of these areas the basis weight of the absorbent material is less than 25%, in particular less than 20%, less than 10%, of the average basis weight of the absorbent material in the rest of the core. In particular there can be no absorbent material in these areas. Minimal amount such as involuntary contaminations with absorbent material that may occur during the making process are not considered as absorbent material. The areas 26 are advantageously surrounded by the absorbent material, when seen in the plane of the core, which means that the area(s) 26 does not extend to any of the edge of the deposition area 8 of the absorbent material.

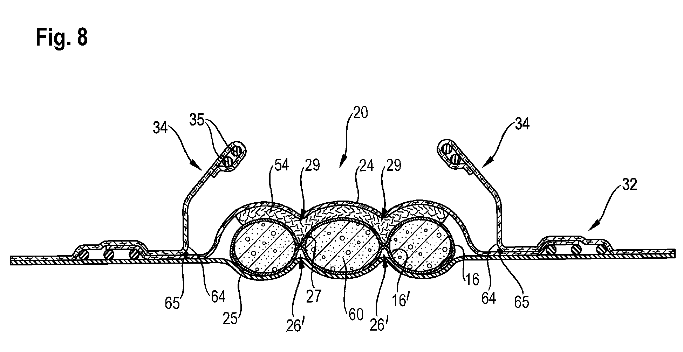

The top side 16 of the core wrap is attached to the bottom side 16' of the core wrap by core wrap bond(s) 27 through these area(s) 26 substantially free of absorbent material. As shown in Fig. 8, when the absorbent material swells upon absorbing a liquid, the core wrap bond remains at least initially attached in the substantially material free area(s) 26. The absorbent material swells in the rest of the core when it absorbs a liquid, so that the core wrap forms one or more channel(s) 26' along the area(s) 26 substantially free of absorbent material comprising the core wrap bond 27. These channels 26' are three dimensional and can serve to distribute an insulting fluid along their length to a wider area of the core. This may provide a quicker fluid acquisition speed and a better utilization of the absorbent capacity of the core. The channels 26' can also provide a deformation of an overlying layer such as a fibrous layer 54 and provide corresponding ditches 29 in the overlying layer. It is not excluded that the absorbent core may comprise other area(s) substantially free of absorbent material but without a core wrap bond, but these non-bonded areas will typically not form a channel when wet.

The top side 16 and the bottom side 16' of the core wrap maybe attached together continuously along the area(s) 26 substantially free of absorbent material, but the core wrap bond 27 may also be discontinuous (intermittent) such as series of point bonds. Typically, an adhesive can be used to attach the top side to the bottom of the core wrap, but it is possible to bond via other known attachment means, such as pressure bonding, ultrasonic bonding or heat bonding or combination thereof. The attachment of the top side and bottom side of the core wrap may be provided by one or more adhesive material, in particular one or more layers of auxiliary glue 71, 72 and/or one or more layers of fibrous adhesive material 51, if present in the core, as indicated below. These glues may therefore serve the dual function of immobilizing the absorbent material and attach the top side and the bottom side of the core together.

The following examples of the shape and size of the areas 26 substantially free of absorbent material are not limiting. In general, the core wrap bond 27 may have the same outline but be slightly smaller than the areas 26 due to the tolerance required in some manufacturing process. The substantially material free area(s) 26 may be present within the crotch region of the article, in particular at least at the same longitudinal level as the crotch point C', as represented in Fig. 1 by the two longitudinally extending areas substantially free of absorbent material 26. The absorbent core 28 may also comprise more than two substantially absorbent material free area(s), for example at least 3, or at least 4 or at least 5 or at least 6. The absorbent core may comprise one or more pairs of areas substantially free of absorbent material symmetrically arranged relative to the longitudinal axis 80'. Shorter area(s) substantially free of absorbent material may also be present, for example in the back region or the front region of the core, as seen for example in the Figures of WO2012/170778.

The area(s) 26 substantially free of absorbent material may extend substantially longitudinally, which means typically that each area extends more in the longitudinal direction than in the transverse direction, and typically at least twice as much in the longitudinal direction than in the transverse direction (as measured after projection on the respective axis). The area(s) 26 substantially free of absorbent material may have a length L' projected on the longitudinal axis 80' of the core that is at least 10% of the length L" of the absorbent core, in particular from 20% to 80%. It may be advantageous that at least some or all of the area(s) 26 are not completely or substantially completely transversely oriented channels in the core.

The area(s) 26 substantially free of absorbent material may be completely oriented longitudinally and parallel to the longitudinal axis but also may be curved. In particular some or all these area(s), in particular these area(s) present in the crotch region, may be concave towards the longitudinal axis 80', as for example represented in Fig. 1 for the pair of channels 26'. The radius of curvature may typically be at least equal (and preferably at least 1.5 or at least 2.0 times this average transverse dimension) to the average transverse dimension of the absorbent material deposition area 8; and also straight but under an angle of (e.g. from 5°) up to 30°, or for example up to 20°, or up to 10° with a line parallel to the longitudinal axis. The radius of curvature may be constant for a substantially absorbent material free area(s), or may vary along its length. This may also includes area(s) substantially free of absorbent material with an angle therein, provided said angle between two parts of a channel is at least 120°, preferably at least 150°; and in any of these cases, provided the longitudinal extension of the area is more than the transverse extension. These area(s) may also be branched, for example a central substantially material free area superposed with the longitudinal axis in the crotch region which branches towards the back and/or towards the front of the article.

In some embodiments, there is no area(s) substantially free of absorbent material that coincides with the longitudinal axis 80' of the core. When present as one ore symmetrical pair(s) relative to the longitudinal axis, the area(s) substantially free of absorbent material may be spaced apart from one another over their whole longitudinal dimension. The smallest spacing distance maybe for example at least 5 mm, or at least 10 mm, or at least 16 mm.

Furthermore, in order to reduce the risk of fluid leakages, the area(s) substantially free of absorbent material may advantageously not extend up to any of the edges of the absorbent material deposition area 8, and are therefore surrounded by and fully encompassed within the absorbent material deposition area 8 of the core. Typically, the smallest distance between an area(s) substantially free of absorbent material and the closest edge of the absorbent material deposition area is at least 5 mm.

The area(s) substantially free of absorbent material may have a width Wc along at least part of its length which is at least 2 mm, or at least 3 mm or at least 4 mm, up to for example 20 mm, or 16 mm or 12 mm. The width Wc of the area(s) substantially free of absorbent material may be constant through substantially its whole length or may vary along its length.

The channels 26' in the absorbent core start forming when the absorbent material absorbs a liquid such as urine and starts swelling. As the core absorbs more liquid, the depressions within the absorbent core formed by channels will become deeper and more apparent to the eye and the touch. It is possible to create a sufficiently strong core wrap bond combined with a relatively low amount of SAP so that the channels remain permanent until complete saturation of the absorbent material. On the other hand, the core wrap bonds may in some cases also restrict the swelling of the absorbent material when the core is substantially loaded.

Initially, the core wrap bond(s) maybe designed to be closed and to increase the pressure in the areas adjacent to the core wrap bond(s). At some point, the core wrap bond (27) may also be designed to open in a controlled manner when exposed to a large amount of fluid.

When the superabsorbent polymer particles have a relatively high value of Absorption Against Pressure (AAP), the force exerted by the superabsorbent polymer particles when the particles swell upon absorbing a liquid, on the core wrap bond(s) 27 may be relatively high. Thus, this force exerted may open the core wrap bond(s) 27 in a controlled manner and may improve the performance of the absorbent core 28 comprising superabsorbent polymer particles as the superabsorbent polymer particles can continue swelling and absorbing fluid.

Conversely, when the superabsorbent polymer particles have a relatively low value of Absorption Against Pressure, the force exerted by the superabsorbent polymer particles when the particles swell upon absorbing, on the core wrap bond(s) 27 may not be sufficient to allow the core wrap bond(s) 27 to open in a controlled manner.

The bonds may thus remain substantially intact at least during a first phase as the absorbent material absorbs a moderate quantity of fluid. In a second phase the core wrap bonds 27 in the channels can start opening to provide more space for the absorbent material to swell while keeping most of the benefits of the channels such as increased flexibility of the core in transversal direction and fluid management. In a third phase, corresponding to a very high saturation of the absorbent core, a more substantial part of the channel bonds can open to provide even more space for the swelling absorbent material to expand. The strength of core wrap bond 27 within the channels can be controlled for example by varying the amount and nature of the glue used for the attaching the two sides of the core wrap, the pressure used to make the core wrap bond and/or the distribution of the absorbent material, as more absorbent material will usually causes more swelling and will put more pressure on the bond.

### Absorbent material deposition area 8

The absorbent material deposition area 8 can be defined by the periphery of the layer formed by the absorbent material 60 within the core wrap, as seen from the top side of the absorbent core. The absorbent material deposition area 8 can be generally rectangular, for example as shown in Fig. 1, but other shapes can also be used such as a "T" or "Y" or "sand-hour" or "dog-bone" shape. In particular the deposition area may which show a tapering along its width towards the middle or "crotch" region of the core. In this way, the absorbent material deposition area may have a relatively narrow width in an area of the core intended to be placed in the crotch region of the absorbent article. This may provide for example better wearing comfort. The absorbent material deposition area 8 may thus have a width (as measured in the transversal direction) at its narrowest point which is less than about 100 mm, 90 mm, 80 mm, 70 mm, 60 mm or even less than about 50 mm. This narrowest width may further be for example at least 5 mm, or at least 10 mm, smaller than the width of the deposition area at its largest point in the front and / or back regions of the deposition area 8.

The basis weight (amount deposited per unit of surface) of the SAP may also be varied along the deposition area 8 to create a profiled distribution of absorbent material, in particular SAP, in the longitudinal direction (as shown in Fig. 4), in the transversal direction, or both directions of the core. Hence along the longitudinal axis of the core, the basis weight of absorbent material may vary, as well as along the transversal axis, or any axis parallel to any of these axes. The basis weight of SAP in area of relatively high basis weight may thus be for example at least 10%, or 20%, or 30%, or 40%, or 50% higher than in an area of relatively low basis weight. In particular the SAP present in the absorbent material deposition area at the longitudinal position of the crotch point C' may have more SAP per unit of surface deposited as compared to another area of the absorbent material deposition area 8.

The absorbent material may be deposited using known techniques, which may allow relatively precise deposition of SAP at relatively high speed. In particular the SAP printing technology as disclosed for example in US2006/024433 (Blessing), US2008/0312617 and US2010/0051166A1 (both to Hundorf et al.) may be used. This technique uses a transfer device such as a printing roll to deposit SAP onto a substrate disposed on a grid of a support which may include a plurality of cross bars extending substantially parallel to and spaced from one another so as to form channels extending between the plurality of cross-bars. This technology allows high-speed and precise deposition of SAP on a substrate in particular to provide one or more area(s) 26 substantially free of absorbent material surrounded by absorbent material. The areas substantially free of absorbent material can be formed for example by modifying the pattern of the grid and receiving drums so that no SAP is applied in the selected areas, as exemplary disclosed in US2012/0312491 (Jackels).

**Microfiber glue 51**

The absorbent core may also comprise a fibrous thermoplastic adhesive material 51, in particular a microfiber glue, to further immobilize the absorbent material within the core. The fibrous thermoplastic adhesive material 51 may be useful to immobilize the layer of absorbent materials 61, 62 to their respective substrate, in particular when the absorbent layer(s) comprises land areas separated by junction areas. The fibrous thermoplastic adhesive material 51 may then be at least partially in contact with the absorbent material 61, 62 in the land areas and at least partially in contact with the substrate layer 16, 16' in the junction areas. This imparts an essentially three-dimensional net-like structure to the fibrous layer of thermoplastic adhesive material 51, which in itself is essentially a two-dimensional structure of relatively small thickness, as compared to the dimension in length and width directions. Thereby, the fibrous thermoplastic adhesive material may provide cavities to cover the absorbent material in the land areas, and thereby immobilizes this absorbent material. The microfiber glue 51 may be for example applied by spraying each absorbent layer.

The thermoplastic polymer may typically have a molecular weight (Mw) of more than 10,000 and a glass transition temperature (Tg) usually below room temperature or -6 °C < Tg < 16 °C. Typical concentrations of the polymer in a hotmelt are in the range of about 20 to about 40% by weight. The thermoplastic polymers may be water insensitive. Exemplary polymers are (styrenic) block copolymers including A-B-A triblock structures, A-B diblock structures and (A-B)n radial block copolymer structures wherein the A blocks are non-elastomeric polymer blocks, typically comprising polystyrene, and the B blocks are unsaturated conjugated diene or (partly) hydrogenated versions of such. The B block is typically isoprene, butadiene, ethylene/butylene (hydrogenated butadiene), ethylene/propylene (hydrogenated isoprene), and mixtures thereof. Other suitable thermoplastic polymers that may be employed are metallocene polyolefins, which are ethylene polymers prepared using single-site or metallocene catalysts. Therein, at least one comonomer can be polymerized with ethylene to make a copolymer, terpolymer or higher order polymer. Also applicable are amorphous polyolefins or amorphous polyalphaolefins (APAO) which are homopolymers, copolymers or terpolymers of C2 to C8 alpha olefins.

The tackifying resin may exemplarily have a Mw below 5,000 and a Tg usually above room temperature, typical concentrations of the resin in a hotmelt are in the range of about 30 to about 60%, and the plasticizer has a low Mw of typically less than 1,000 and a Tg below room temperature, with a typical concentration of about 0 to about 15%.

The thermoplastic adhesive used for the fibrous layer preferably has elastomeric properties, such that the web formed by the fibers on the SAP layer is able to be stretched as the SAP swell. Exemplary elastomeric, hotmelt adhesives include thermoplastic elastomers such as ethylene vinyl acetates, polyurethanes, polyolefin blends of a hard component (generally a crystalline polyolefin such as polypropylene or polyethylene) and a Soft component (such as ethylene-propylene rubber); copolyesters such as poly (ethylene terephthalate-co-ethylene azelate); and thermoplastic elastomeric block copolymers having thermoplastic end blocks and rubbery mid blocks designated as A-B-A block copolymers: mixtures of structurally different homopolymers or copolymers, e.g., a mixture of polyethylene or polystyrene with an A-B-A block copolymer; mixtures of a thermoplastic elastomer and a low molecular weight resin modifier, e.g., a mixture of a styrene-isoprenestyrene block copolymer with polystyrene; and the elastomeric, hot-melt, pressure-sensitive adhesives described herein. Elastomeric, hot-melt adhesives of these types are described in more detail in U.S. 4,731,066 (Korpman).

The thermoplastic adhesive material 51 fibers may exemplarily have an average thickness of about 1 to about 50 micrometers or about 1 to about 35 micrometers and an average length of about 5 mm to about 50 mm or about 5mm to about 30 mm. To improve the adhesion of the thermoplastic adhesive material to the substrate or to any other layer, in particular any other nonwoven layer, such layers may be pre-treated with an auxiliary adhesive. The fibers adhere to each other to form a fibrous layer, which can also be described as a mesh.

The absorbent core advantageously achieve an SAP loss of no more than about 70%, 60%, 50%, 40%, 30%, 20%, 10% according to the Wet Immobilization Test described in US2010/0051166A1.

**Auxiliary glue 71, 72**

The absorbent core of the invention may further comprise an auxiliary glue present on the inner surface of the top side and/ bottom side of the absorbent core, in particular to help immobilizing the SAP within the core wrap, to ensure integrity of the core wrap and/or to form the bond 27 attaching the bottom side of the core wrap to the top side of the core wrap through the one or more area(s) substantially free of absorbent material.

This so-called auxiliary glue 71, 72 can be applied on the inner surface of the top side and/or the bottom side of the core wrap. The auxiliary glue may be any conventional glue used in the field, in particular hotmelt glue. Example of glues are based on an adhesive polymer such SIS (Styrene-Isoprene-Block Co-Polymer), SBS (Styrene-Butadiene-Block Co-polymer) or mPO (metalocine Polyolefine). The glue may also comprise a tackifier such as a hydrogenated hydrocarbon resin, as well as an oil and an antioxidant. Hydrogenated hydrocarbon resins are made from mixed aromatic/aliphatic resins which are subsequently selectively hydrogenated to produce a wide range of materials with low color, high stability and broad compatibility. Examples of commercially available adhesives are available as HL1358LO and NW1286 (both from HB Fuller) and DM 526 (from Henkel).

The auxiliary glue may be applied on the top side and/or the bottom side of the core wrap in an average amount ranging from 2 gsm to 20 gsm, more particularly from 4 gsm to 10 gsm. The auxiliary glue may be uniformly applied, or discontinuously, in particular as a series of stripes regularly spaced and longitudinally oriented, for example a series of auxiliary glue stripes of about 1 mm width spaced from each other by a distance raging from 1 mm to 3 mm. The auxiliary glue may help forming the core wrap bond 27 if sufficient pressure and glue is applied within the material free area 26 to attach both sides of the core wrap. The auxiliary glue layer may be applied to the inner surface of the bottom side, the inner surface of the top side, or both inner surfaces of the core wrap.

## General description of the absorbent article

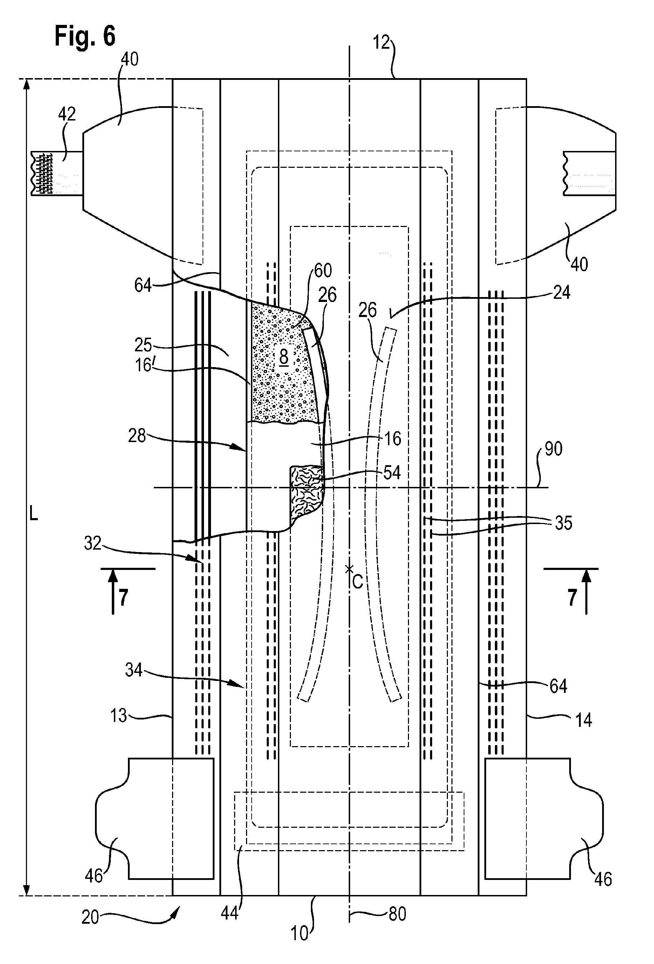

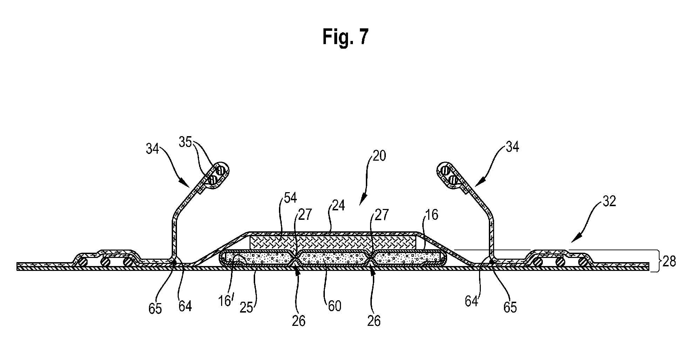

Having now discussed in quite details certain embodiments of the absorbent cores of the invention, the absorbent articles in which these cores may be used will now be generally discussed and further illustrated in the form of a baby diaper 20 in Figs. 6-8. Fig. 6 is a plan view of the exemplary diaper 20, in a flattened state, with portions of the structure being cut-away to more clearly show the construction of the diaper 20. This diaper 20 is shown for illustration purpose only as the invention may be used for making a wide variety of diapers or other absorbent articles.

The absorbent article comprises a liquid permeable topsheet 24, a liquid impermeable backsheet 25, and an absorbent core 28 between the topsheet 24 and the backsheet 25. An optional acquisition / distribution layer 54 is represented on Fig. 6, which also shows other typical taped diaper components such as a fastening system comprising adhesive tabs 42 attached towards the back edge of the article and cooperating with a landing zone 44 on the front of the article, barrier leg cuffs 34 and elasticized gasketing cuffs 32 joined to the chassis of the absorbent article, typically via the topsheet and/or backsheet, and substantially planar with the chassis of the diaper. The absorbent article may also comprise other typical elements, which are not represented, such as a back elastic waist feature, a front elastic waist feature, transverse barrier cuff(s), a lotion application, etc.

The barrier leg cuffs 34 can be formed from a piece of material, typically a nonwoven, which is partially bonded to the rest of the article so that a portion of the material, the barrier leg cuffs, can be partially raised away and stand up from the plane defined by the topsheet when the article is pulled flat as shown e.g. in Fig. 6. The barrier leg cuffs can provide improved containment of liquids and other body exudates approximately at the junction of the torso and legs of the wearer. The barrier leg cuffs extend at least partially between the front edge and the back edge of the diaper on opposite sides of the longitudinal axis and are at least present at the longitudinal position of the crotch point (C). The barrier leg cuffs are delimited by a proximal edge 64 joined to the rest of the article, typically the topsheet and/or the backsheet, and a free terminal edge , which is intended to contact and form a seal with the wearer's skin. The barrier leg cuffs are joined at the proximal edge 64 with the chassis of the article by a bond 65 which may be made for example by gluing, fusion bonding or combination of known bonding means. The bond 65 at the proximal edge 64 may be continuous or intermittent. The side of the bond 65 closest to the raised section of the barrier leg cuffs 32 delimits the proximal edge 64 of the standing up section of the leg cuffs. Each barrier leg cuff 34 may comprise one, two or more elastic strings 35 close to this free terminal edge to provide a better seal.

The absorbent article may comprise front ears 46 and back ears 40 as it is known in the art. The back ears 40 are advantageously stretchable to facilitate the attachment of the tabs 42 on the landing zone 44 and maintain the taped diapers in place around the wearer's waist.

The absorbent article 20 comprises a front edge 10, a back edge 12, and two side (longitudinal edges) 13, 14. The front edge 10 of the article is the edge which is intended to be placed towards the front of the user when worn, and the back edge 12 is the opposite edge of the article. The absorbent article may be notionally divided by a longitudinal axis 80 extending from the front edge to the back edge of the article and dividing the article in two substantially symmetrical halves relative to this axis, with article placed flat and viewed from above as in Fig. 6. The length L of the article can be measured along the longitudinal axis 80 from front edge 10 to back edge 12. The article comprises a crotch point C defined herein as the point placed on the longitudinal axis at a distance of two fifth (2/5) of L starting from the front edge 10 of the article 20. The width of the article for a diaper application at the crotch point may in particular be of from 50 mm to 300 mm, or from 80 mm to 250 mm. For adult incontinence products the width may go up to 450 mm.

The crotch region can be defined as the region of the diaper longitudinally centered at the crotch point C and extending towards the front and towards the back of the absorbent article by a distance of one fifth of L (L/5) in each direction. A front region and a back region can be defined as the remaining portions of the diapers placed respectively towards the front and the back edges of the article.

The topsheet 24, the backsheet 25, the absorbent core 28 and the other article components may be assembled in a variety of well known configurations, in particular by gluing or heat embossing. Exemplary diaper configurations are described generally in US3,860,003, US5,221,274, US5,554,145, US5,569,234, US5,580,411, and US6,004,306.

For most absorbent articles, the liquid discharge occurs predominately in the front half of the article, in particular for diaper. The front half of the article (as defined by the region between the front edge and a transversal line 90 placed at a distance of half L from the front or back edge may therefore comprise most of the absorbent capacity of the core. Thus, at least 60% of the absorbent material, or at least 65%, or at least 70%, or at least 75% or at least 80% of the absorbent material may be present in the front half of the absorbent article, the remaining absorbent material being disposed in the back half of the absorbent article.

The basis weight of superabsorbent polymer particles in the absorbent material may vary along the absorbent article. The superabsorbent polymer particles in the absorbent material may have a higher basis weight in the crotch region compared to the front region and the back region. Alternatively, the superabsorbent polymer particles in the absorbent material may have a higher basis weight in the front region or the back region compared to the crotch region.

### Topsheet

The topsheet 24 is the layer of the absorbent article that is destined to be in contact with the wearer's skin. The topsheet 24 can be joined to the backsheet 25, the core 28 and/or any other layers as is known in the art. Usually, the topsheet 24 and the backsheet 25 may be joined directly to each other on or close to the periphery of the article and are indirectly joined together in other locations by directly joining them to one or more other elements of the article 20. The topsheet may be attached to an underlying layer 54, which may be an acquisition and/or distribution layer, by any conventional means, in particular gluing, mechanical or heat bonding and combinations thereof. The topsheet may in particular be attached directly or indirectly to the fibrous layer 54 in the area where the ditches of the fibrous layer are formed, as exemplarily shown in Fig. 8. This may provide or help the formation of secondary ditches 29 at the surface of the article.

The topsheet 24 is preferably compliant, soft-feeling, and non-irritating to the wearer's skin. Further, at least a portion of the topsheet 24 is liquid permeable, permitting liquids to readily penetrate through its thickness. A suitable topsheet may be manufactured from a wide range of materials, such as porous foams, reticulated foams, apertured plastic films, or woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. If the topsheet includes fibers, the fibers may be spunbond, carded, wet-laid, meltblown, hydroentangled, or otherwise processed as is known in the art, in particular spunbond PP nonwoven. A suitable topsheet comprising a web of staple-length polypropylene fibers is manufactured by Veratec, Inc., a Division of International Paper Company, of Walpole, MA under the designation P-8.

Any portion of the topsheet 24 maybe coated with a lotion as is known in the art. The topsheet 24 may also include or be treated with antibacterial agents. Further, the topsheet 24, the backsheet 25 or any portion of the topsheet or backsheet may be embossed and/or matte finished to provide a more cloth like appearance.

The topsheet 24 may comprise one or more apertures to ease penetration of exudates therethrough, such as urine and/or feces (solid, semi-solid, or liquid). Typically, the total area of the apertures at the surface of a diaper may have an area of between about 10 cm2 and about 50 cm2, in particular between about 15 cm2 and 35 cm2.

Typical diaper topsheets have a basis weight of from about 10 to about 28 gsm, in particular between from about 12 to about 18 gsm but other basis weights are possible.

### Backsheet

The backsheet 25 is generally that portion of the absorbent article 20 which forms the majority of the external surface of the article when worn by the user. The backsheet is positioned towards the bottom side of the absorbent core and prevents the exudates absorbed and contained therein from soiling articles such as bedsheets and undergarments. The backsheet 25 is typically impermeable to liquids (e.g. urine). The backsheet may for example be or comprise a thin plastic film such as a thermoplastic film having a thickness of about 0.012 mm to about 0.051 mm. Other suitable backsheet materials may include breathable materials which permit vapors to escape from the diaper 20 while still preventing exudates from passing through the backsheet 25. Exemplary breathable materials may include materials such as woven webs, nonwoven webs, composite materials such as film-coated nonwoven webs, microporous films, and monolithic films.

The backsheet 25 may be joined to the topsheet 24, the absorbent core 28 or any other element of the diaper 20 by any attachment means known in the art. Suitable attachment means are described above with respect to means for joining the topsheet 24 to other elements of the article 20. For example, the attachment means may include a uniform continuous layer of adhesive, a patterned layer of adhesive, or an array of separate lines, spirals, or spots of adhesive. Alternatively, the attachment means may comprise heat bonds, pressure bonds, ultrasonic bonds, dynamic mechanical bonds, or any other suitable attachment means or combinations of these attachment means as are known in the art.

**Additional layer**

The absorbent article may further comprise one or more additional layer 54 that can serve to acquire and distribute the fluid, as illustrate by layer 54 in the Figures. The additional layer(s) may be present between the topsheet 24 and the absorbent core 28, as represented in the Figures, but it maybe also between the backsheet 25 and the absorbent core 28, or both. The additional layer 54 maybe at least partially bonded to the top side or the bottom side of the core wrap in the area(s) substantially free of absorbent material. The formation of the channel 26' in the absorbent core as the absorbent material swells may thus provides of one or more corresponding ditches 29 in the additional layer 54.

The additional layer(s) may be of any kind such as nonwoven, a woven material or even loose fibers. The additional layers may in particular be of the type known in the art for acquisition layers and/or distribution layers.

A distribution layer can spread an insulting fluid liquid over a larger surface within the article so that the absorbent capacity of the core can be more efficiently used. Typically distribution layers are made of a nonwoven material based on synthetic or cellulosic fibers and having a relatively low density. The density of the distribution layer may vary depending on the compression of the article, but may typically range from 0.03 to 0.25 g/cm3, in particular from 0.05 to 0.15 g/cm3 measured at 0.30 psi (2.07kPa). The distribution layer may also be a material having a water retention value of from 25 to 60, preferably from 30 to 45, measured as indicated in the procedure disclosed in US5,137,537. The distribution layer may typically have an average basis weight of from 30 to 400 g/m2, in particular from 100 to 300 g/m2.

The distribution layer may for example comprise at least 50% by weight of cross-linked cellulose fibers. The cross-linked cellulosic fibers may be crimped, twisted, or curled, or a combination thereof including crimped, twisted, and curled. This type of material has been used in the past in disposable diapers as part of an acquisition system, for example US 2008/0312622 A1 (Hundorf). The cross-linked cellulosic fibers provide higher resilience and therefore higher resistance to the first absorbent layer against the compression in the product packaging or in use conditions, e.g. under a baby's weight. This provides the core with a higher void volume, permeability and liquid absorption, and hence reduced leakage and improved dryness.

Exemplary cross-linking agents include polycarboxylic acids such as citric acid and/or polyacrylic acids such as acrylic acid and maleic acid copolymers.

The absorbent article may also comprise an acquisition layer as additional layer, whose function can be to quickly acquire the fluid away from the topsheet so as to provide a good dryness for the wearer. Such an acquisition layer is typically placed directly under the topsheet. The absorbent article may also then comprise a distribution layer typically placed between the acquisition layer and the absorbent core.

The acquisition layer may typically be or comprise a non-woven material, for example a SMS or SMMS material, comprising a spunbonded, a melt-blown and a further spunbonded layer or alternatively a carded chemical-bonded nonwoven. The non-woven material may in particular be latex bonded. Carded, resin-bonded nonwovens may be used, in particular where the fibers used are solid round or round and hollow PET staple fibers (50/50 or 40/60 mix of 6 denier and 9 denier fibers). An exemplary binder is a butadiene/styrene latex. Non-wovens have the advantage that they can be manufactured outside the converting line and stored and used as a roll of material.

Such an acquisition layer 54 may be stabilized by a latex binder, for example a styrene-butadiene latex binder (SB latex).

A further acquisition layer may be used in addition to a first acquisition layer described above. For example a tissue layer may be placed between the first acquisition layer and the distribution layer. The tissue may have enhanced capillarity distribution properties compared to the acquisition layer described above. The tissue and the first acquisition layer may be of the same size or may be of different size, for example the tissue layer may extend further in the back of the absorbent article than the first acquisition layer. An example of hydrophilic tissue is a 13 - 22.5 gsm high wet strength made of cellulose fibers from supplier Havix.

If an acquisition layer is present, it may be advantageous that this acquisition layer is larger than or least as large as an underlying distribution layer in the longitudinal and/or transversal dimension. In this way the distribution layer can be deposited on the acquisition layer. This simplifies handling, in particular if the acquisition layer is a nonwoven which can be unrolled from a roll of stock material. The distribution layer may also be deposited directly on the absorbent core's upper side of the core wrap or another layer of the article. Also, an acquisition layer larger than the distribution layer allows to directly glue the acquisition layer to the storage core (at the larger areas). This can give increased patch integrity and better liquid communication.

**Method of making the article - Relations between the layers**

The absorbent articles of the invention may be made by any conventional methods known in the art. Typically, adjacent layers and components will be joined together using conventional bonding method such as adhesive coating via slot coating or spraying on the whole or part of the surface of the layer, or thermo-bonding, or pressure bonding or combinations thereof. This bonding is exemplarily represented for the bond between the leg cuffs 65 and the topsheet 24 on Fig. 6. Other glues or attachments are not represented for clarity and readability but typical bonding between the layers of the article should be considered to be present unless specifically excluded. Adhesives may be typically used to improve the adhesion of the different layers, for example between the backsheet and the core wrap. The glue may be any standard hotmelt glue as known in the art.

The absorbent core and in particular its absorbent material deposition area 8 may advantageously be at least as large and long and advantageously at least partially larger and/or longer than the fibrous layer. This is because the absorbent material in the core can usually more effectively retain fluid and provide dryness benefits across a larger area than the fibrous layer. The absorbent article may have a rectangular absorbent material layer and a non-rectangular (shaped) fibrous layer. The absorbent article may also have a rectangular (non-shaped) fibrous layer and a rectangular layer of absorbent material.

**Packages**

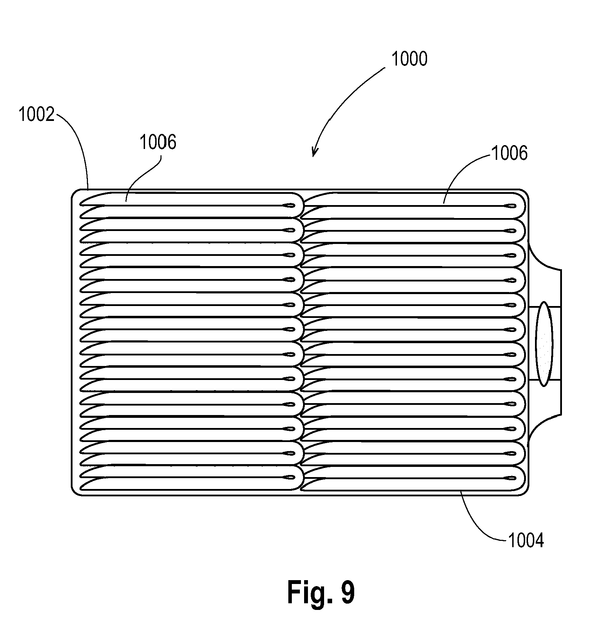

The articles may be folded and packaged as is known in the art. The package may be for example a plastic bag or a cardboard box. Diapers may typically bi-folded along the transversal axis and the ears folded inwardly before being packaged. The absorbent articles may be packed under compression so as to reduce the size of the packages, while still providing an adequate amount of absorbent articles per package. By packaging the absorbent articles under compression, caregivers can easily handle and store the packages, while also providing distribution and inventory savings to manufacturers owing to the size of the packages. Fig. 9 illustrates an example package 1000 comprising a plurality of absorbent articles 1004. The package 1000 defines an interior space 1002 in which the plurality of absorbent articles 1004 are situated. The plurality of absorbent articles 1004 are arranged in one or more stacks 1006.

The three-dimensional material may be particularly resilient to compression so that the articles may be compressed to a certain extent in the package. It is believed that the plurality of relatively closely spaced, relatively small, and relatively pillowy three-dimensional projections may act as springs to resist compression and recover once a compressive force is removed, especially in the areas in the vicinity of the channels. Compression recovery is important in nonwoven or other component layers of absorbent articles, because such articles are typically packaged and folded in compressed conditions. Manufacturers of personal care products desire to retain most, if not all of the as-made caliper for aesthetic and performance purposes. Furthermore, it is believed the channels being substantially material-free may contribute to an unexpected, beneficial improvement in compression recovery as they provide spacing for at some of the three-dimensional projections to nest in during storage and transport in the compressed package state

The articles of the inventions may thus be packaged compressed at an In-Bag Compression Rate of at least 10%, in particular of from 10% to 50%, in particular from 20% to 40%. The "In-Bag Compression Rate" as used herein is one minus the height of a stack of 10 folded articles measured while under compression within a bag ("In-Bag Stack Height") divided by the height of a stack of 10 folded articles of the same type before compression, multiplied by 100; i.e. (1-In-Bag Stack Height/stack height before compression) * 100, reported as a percentage. Of course, the stack in the bag does not need to have exactly 10 articles, rather the value measured for the height of stack of article in the package is divided by the number of articles in the stack and then multiplied by 10. The method used to measure the In-Bag Stack Height is described in further details in the Test Procedures. The articles before compression may be typically sampled from the production line between the folding unit and the stack packing unit. The stack height before compression is measured by taking 10 articles before compression and packing, and measuring their stack height as indicated for the IBSH.

Packages of the absorbent articles of the present disclosure may have an In-Bag Stack Height of from 70 mm to 110 mm.

Packages of the absorbent articles of the present disclosure may in particular have an In-Bag Stack Height of less than 110 mm, less than 105 mm, less than 100 mm, less than 95 mm, less than 90 mm, specifically reciting all 0.1 mm increments within the specified ranges and all ranges formed therein or thereby, according to the In-Bag Stack Height Test described herein. For the values shared in the previous sentence, it may be desirable to have an In-Bag Stack Height of greater than 70 mm, or greater than 75 mm, or greater than 80 mm. Alternatively, packages of the absorbent articles of the present disclosure may have an In-Bag Stack Height of from 70 mm to 110 mm, from 75 mm to 110 mm, from 80 mm to 110 mm, from 80 mm to 105 mm, or from 80 mm to 100 mm, specifically reciting all 0.1 mm increments within the specified ranges and all ranges formed therein or thereby, according to the In-Back Stack Height Test described herein.

**Test Method:**

**Centrifuge Retention Capacity (CRC) test method**

The CRC measures the liquid absorbed by the superabsorbent polymer particles for free swelling in excess liquid. The CRC is measured according to EDANA method WSP 241.2-05.

**Absorption Against Pressure (AAP) test method**

The AAP is measured according to EDANA method WSP 442.2-02.

**Bulk density test method**

The bulk density test method refers to the EDANA method WSP 460.2-02.

**Effective Capacity (EFFC)**

The Effective Capacity represents an average of the value of Centrifuge Retention Capacity (CRC) and of the value of Absorption Against Pressure (AAP) of the superabsorbent polymer particles.
The Effective Capacity (EFFC) is calculated via the formula below: EFFC = (CRC+AAP)/2.

**Urine Permeability Measurement Test method (UPM):**

The UPM is measured according to UPM Test method set out in WO2012/174,026A1.

**Dry Absorbent Core Caliper Test**

This test may be used to measure the caliper of the absorbent core (before use i.e. without fluid loading) in a standardized manner at the crotch point C' of the core or any other point.
Equipment: Mitutoyo manual caliper gauge with a resolution of 0.01 mm -- or equivalent instrument.
Contact Foot: Flat circular foot with a diameter of 17.0 mm ($\pm$ 0.2 mm). A circular weight may be applied to the foot (e.g., a weight with a slot to facilitate application around the instrument shaft) to achieve the target weight. The total weight of foot and added weight (including shaft) is selected to provide 2.07 kPa (0.30 psi) of pressure to the sample.
The caliper gauge is mounted with the lower surface of the contact foot in an horizontal plane so that the lower surface of the contact foot contacts the center of the flat horizontal upper surface of a base plate approximately 20 x 25 cm. The gauge is set to read zero with the contact foot resting on the base plate.

Ruler: Calibrated metal ruler graduated in mm.
Stopwatch: Accuracy 1 second
Sample preparation: The core is conditioned at least 24 hours as indicated above.
Measurement procedure: The core is laid flat with the bottom side, i.e. the side intended to be placed towards the backsheet in the finished article facing down. The point of measurement (e.g. the crotch point C corresponding to this point in the finished article) is carefully drawn on the top side of the core taking care not to compress or deform the core.

The contact foot of the caliper gauge is raised and the core is placed flat on the base plate of the caliper gauge with the top side of the core up so that when lowered, the center of the foot is on the marked measuring point.
The foot is gently lowered onto the article and released (ensure calibration to "0" prior to the start of the measurement). The caliper value is read to the nearest 0.01 mm, 10 seconds after the foot is released.
The procedure is repeated for each measuring point. If there is a fold at the measuring point, the measurement is done in the closest area to this point but without any folds. Ten articles are measured in this manner for a given product and the average caliper is calculated and reported with an accuracy of one tenth mm.

**In-Bag Stack Height Test**

The In-Bag stack height of a package of absorbent articles is determined as follows:

Equipment: A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within $\pm$ 0.5 mm. The horizontal sliding plate and the horizontal base plate are larger than the surface of the absorbent article package that contacts each plate, i.e. each plate extends past the contact surface of the absorbent article package in all directions. The horizontal sliding plate exerts a downward force of 850 $\pm$ 1 gram-force (8.34 N) on the absorbent article package, which may be achieved by placing a suitable weight on the center of the non-package-contacting top surface of the horizontal sliding plate so that the total mass of the sliding plate plus added weight is 850 $\pm$ Igrams. Test Procedure: Absorbent article packages are equilibrated at 23 $\pm$ 2 °C and 50 $\pm$ 5% relative humidity prior to measurement. The horizontal sliding plate is raised and an absorbent article package is placed centrally under the horizontal sliding plate in such a way that the absorbent articles within the package are in a horizontal orientation. Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within $\pm$0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) x 10 is calculated and reported to within $\pm$0.5 mm.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

**Claims**

1. An absorbent core (28) for use in an absorbent article comprising:

a core wrap (16, 16') enclosing an absorbent material (60), the absorbent material comprising superabsorbent polymer particles,
wherein the core wrap comprises a top side (16) and a bottom side (16'),
wherein the absorbent core comprises one or more area(s) (26) substantially free of absorbent material through which the top side of the core wrap is attached to the bottom side of the core wrap, so that when the absorbent material swells the core wrap forms one or more channel(s) (26') along the area(s) substantially free of absorbent material,
**characterized in that** the superabsorbent polymer particles have a value of Effective Capacity (EFFC) of at least 27 g/g;
wherein The Effective Capacity (EFFC) is calculated via the formula below:

$$EFFC = (CRC+AAP)/2;$$

wherein the Centrifuge Retention Capacity (CRC) is measured according to the Centrifuge Retention Capacity (CRC) test method and the Absorption Against Pressure (AAP) is measured according to the Absorption Against Pressure (AAP) test method; and

wherein the superabsorbent polymer particles have a bulk density of at least 0.5 g/ml according to the Bulk Density Test Method.

2. The absorbent core according to claim 1 wherein the value of EFFC is between 27 g/g and 32 g/g.

3. The absorbent core according to any of the preceding claims, wherein the bulk density of the superabsorbent polymer particles is at least 0.6 g/ml.

4. The absorbent core according to any of the preceding claims, wherein the superabsorbent polymer particles have a value of absorption against pressure (AAP) of at least 22 g/g.

5. The absorbent core according to any of the preceding claims, wherein the absorbent material comprises at least 80%, in particular at least 90%, more particularly at least 95% of superabsorbent polymer particles by weight of the absorbent material.

6. The absorbent core according to any of the preceding claims wherein the absorbent material comprises less than 10% by weight of fibrous absorbent material, or less than 5% by weight of fibrous absorbent material, or is substantially free of fibrous absorbent material.

7. The absorbent core according to any of the preceding claims wherein the superabsorbent polymer particles have a permeability at equilibrium expressed as UPM (Urine Permeability Measurement) value of more than 20 UPM units according to the UPM Test method.

8. The absorbent core according to any of the preceding claims wherein the superabsorbent polymer particles have a permeability at equilibrium expressed as UPM (Urine Permeability Measurement) value of more than 65 UPM units according to the UPM Test method.

9. The absorbent core according to any of the preceding claims, wherein at least one of the area(s) (26) substantially free of absorbent material has/have a width (Wc) in at least in some part of the area of at least 2 mm, in particular from 4 mm to 20 mm.

10. The absorbent core according to any of the preceding claims wherein the core wrap comprises a first nonwoven substantially forming the top side (16) of the core wrap and a second nonwoven substantially forming the bottom side (16') of the core wrap, preferably wherein the first nonwoven forms a C-wrap around the second nonwoven.

11. The absorbent core according to any of the preceding claims, wherein the periphery of the absorbent material within the core wrap defines an absorbent material deposition area (8), and wherein the absorbent material deposition area is either rectangular or is shaped with a width narrower at the crotch point (C') than the maximum width of the absorbent material deposition area in the rest of the core, wherein the crotch point is defined as the point placed at a distance of two fifth (2/5) of L" from the front edge (280) of the absorbent core on the longitudinal axis (80') of the core.

12. An absorbent core according to any of the preceding claims comprising a fibrous thermoplastic adhesive material 51, in particular a microfiber glue, to immobilize the absorbent material within the core

13. An absorbent core according to any of the preceding claims further comprising an auxiliary glue between the absorbent material and the top side and/or the bottom side of the core wrap.

14. The absorbent article (20) for personal hygiene comprising:

   a liquid permeable topsheet (24),
   a liquid impermeable backsheet (25),
   optionally an acquisition and /or distribution layer (54), and
   an absorbent core (28) according to any of the preceding claims between the topsheet and backsheet.

15. A package comprising a plurality of the absorbent articles according to any of the preceding claims, wherein the package has an In-Bag Stack Height of from 70 mm to 110 mm.

# Fig. 1

**Fig. 2**

**Fig. 3**

**Fig. 5**

**Fig. 4**

# Fig. 6

# Fig. 7

**Fig. 8**

**Fig. 9**

**EUROPEAN SEARCH REPORT**

Application Number

EP 15 19 4750

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 813 201 A1 (PROCTER & GAMBLE [US]) 17 December 2014 (2014-12-17) * paragraphs [0008], [0023], [0033] - [0044], [0052] - [0054], [0055] - [0062]; figures 1-16 * | 1-15 | INV. A61F13/532 A61F13/534 A61L15/60 A61F13/53 |
| X | US 2014/163503 A1 (ARIZTI BLANCA [DE] ET AL) 12 June 2014 (2014-06-12) * paragraph [0041] - paragraph [0075]; figures 1-8 * | 1-15 | |
| Y | EP 2 781 259 A1 (NIPPON CATALYTIC CHEM IND [JP]) 24 September 2014 (2014-09-24) * paragraph [0249] * | 1-15 | |
| Y | EP 2 270 057 A1 (NIPPON CATALYTIC CHEM IND [JP]) 5 January 2011 (2011-01-05) * paragraph [0122]; claims 26,27 * | 1-15 | |
| Y | WO 2013/056978 A2 (BASF SE [DE]; SCHROEDER ULRICH [DE]; FUNK RUEDIGER [DE]; LOUDEN JOHN J) 25 April 2013 (2013-04-25) * claims 1-15 * | 1-15 | TECHNICAL FIELDS SEARCHED (IPC) A61F A61L |
| Y | GB 2 283 680 A (KIMBERLY CLARK CO [US]) 17 May 1995 (1995-05-17) * claims 1-24; figures 1-14 * | 1-15 | |
| Y | WO 2004/113452 A1 (NIPPON CATALYTIC CHEM IND [JP]; ADACHI YOSHIFUMI; KITANO TAKAHIRO; FUJ) 29 December 2004 (2004-12-29) * claims 1-15 * | 1-15 | |

The present search report has been drawn up for all claims

| Place of search | Date of completion of the search | Examiner |
|---|---|---|
| Munich | 4 April 2016 | Gennari, Silvia |

## ANNEX TO THE EUROPEAN SEARCH REPORT
## ON EUROPEAN PATENT APPLICATION NO.

EP 15 19 4750

This annex lists the patent family members relating to the patent documents cited in  the above-mentioned European search report.
The members are as contained in the European Patent Office EDP file on
The European Patent Office is in no way liable for these particulars which are merely given for the purpose of information.

04-04-2016

| Patent document cited in search report | | Publication date | Patent family member(s) | | Publication date |
|---|---|---|---|---|---|
| EP 2813201 | A1 | 17-12-2014 | CA | 2915216 A1 | 18-12-2014 |
| | | | CN | 105263455 A | 20-01-2016 |
| | | | EP | 2813201 A1 | 17-12-2014 |
| | | | KR | 20160007618 A | 20-01-2016 |
| | | | US | 2014371701 A1 | 18-12-2014 |
| | | | WO | 2014200794 A1 | 18-12-2014 |
| US 2014163503 | A1 | 12-06-2014 | CA | 2894677 A1 | 19-06-2014 |
| | | | CN | 104837458 A | 12-08-2015 |
| | | | EP | 2740452 A1 | 11-06-2014 |
| | | | JP | 2015536230 A | 21-12-2015 |
| | | | US | 2014163503 A1 | 12-06-2014 |
| | | | WO | 2014093319 A1 | 19-06-2014 |
| EP 2781259 | A1 | 24-09-2014 | CN | 103930201 A | 16-07-2014 |
| | | | EP | 2781259 A1 | 24-09-2014 |
| | | | JP | WO2013073614 A1 | 02-04-2015 |
| | | | US | 2014299815 A1 | 09-10-2014 |
| | | | WO | 2013073614 A1 | 23-05-2013 |
| EP 2270057 | A1 | 05-01-2011 | CN | 102015777 A | 13-04-2011 |
| | | | EP | 2270057 A1 | 05-01-2011 |
| | | | JP | 5730013 B2 | 03-06-2015 |
| | | | US | 2011034603 A1 | 10-02-2011 |
| | | | WO | 2009130915 A1 | 29-10-2009 |
| WO 2013056978 | A2 | 25-04-2013 | CN | 103889385 A | 25-06-2014 |
| | | | EP | 2768456 A2 | 27-08-2014 |
| | | | JP | 2014530077 A | 17-11-2014 |
| | | | WO | 2013056978 A2 | 25-04-2013 |
| GB 2283680 | A | 17-05-1995 | AU | 677451 B2 | 24-04-1997 |
| | | | AU | 8018194 A | 22-05-1995 |
| | | | CA | 2117125 A1 | 30-05-1995 |
| | | | DE | 69417051 D1 | 15-04-1999 |
| | | | DE | 69417051 T2 | 23-09-1999 |
| | | | EP | 0725616 A1 | 14-08-1996 |
| | | | ES | 2127947 T3 | 01-05-1999 |
| | | | FR | 2711518 A1 | 05-05-1995 |
| | | | GB | 2283680 A | 17-05-1995 |
| | | | JP | 3434296 B2 | 04-08-2003 |
| | | | JP | H09504207 A | 28-04-1997 |
| | | | KR | 100345267 B1 | 11-10-2003 |
| | | | PH | 30532 A | 27-06-1997 |
| | | | TW | 259701 B | 11-10-1995 |
| | | | US | 5433715 A | 18-07-1995 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**

EP 15 19 4750

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2016

| Patent document cited in search report | Publication date | Patent family member(s) | | Publication date |
|---|---|---|---|---|
| | | WO | 9511652 A1 | 04-05-1995 |
| | | ZA | 9407442 A | 11-05-1995 |
| WO 2004113452 A1 | 29-12-2004 | BR | PI0411370 A | 01-08-2006 |
| | | CN | 1813033 A | 02-08-2006 |
| | | EP | 1641883 A1 | 05-04-2006 |
| | | KR | 20060027360 A | 27-03-2006 |
| | | MX | PA05013945 A | 24-02-2006 |
| | | RU | 2333229 C2 | 10-09-2008 |
| | | TW | I332021 B | 21-10-2010 |
| | | US | 2007106013 A1 | 10-05-2007 |
| | | WO | 2004113452 A1 | 29-12-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5151092 A, Buell
- WO 2008155699 A, Hundorf
- WO 9511652 A, Tanzer
- WO 2012052172 A, Van Malderen
- WO 2012170778 A, Rosati
- WO 2012170779 A
- WO 2012170781 A
- WO 2012170808 A
- US 7744576 B
- US 20110268932 A1
- US 20110319848 A1
- US 20110250413 A1
- WO 02064877 A
- US 7744576 B2, Busam
- US 20080312622 A1
- WO 2006083584 A
- WO 2007047598 A
- WO 2007046052 A
- WO 2009155265 A
- WO 2009155264 A
- WO 2012174026 A1
- US 2006024433 A, Blessing
- US 20080312617 A
- US 20100051166 A1, Hundorf
- US 20120312491 A, Jackels
- US 4731066 A, Korpman
- US 3860003 A
- US 5221274 A
- US 5554145 A
- US 5569234 A
- US 5580411 A
- US 6004306 A
- US 5137537 A

**Non-patent literature cited in the description**

- Contact angle, wettability and adhesion. 1964